# EUROPÄISCHE PATENTSCHRIFT

(11) **EP 1 223 220 B1**
(45) Veröffentlichungstag und Bekanntmachung des Hinweises auf die Patenterteilung: **01.03.2006**
(21) Anmeldenummer: 01130058.9
(22) Anmeldetag: 18.12.2001
(51) Int. Cl.: C12N 15/60, C12N 9/88, C12P 13/00

(54) **Gene, enthaltend eine für Hydroxynitrillyase codierende DNA-Sequenz, rekombinante Proteine mit Hydroxynitrillyase-Aktivität und deren Verwendung**
Genes coding for hydroxynitrile lyase, recombinant proteins with hydroxynitrile lyase activity and their use
Gènes codant pour des hydroxynitrile lyases, protéines récombinantes à activité d'hydroxynitrile lyase et leur utilisation

(30) Priorität: 16.01.2001 AT 602001; 03.04.2001 AT 5232001
(43) Veröffentlichungstag der Anmeldung: 17.07.2002
(60) Teilanmeldung: 05001539.5
(73) Patentinhaber: DSM Fine Chemicals Austria Nfg GmbH & Co KG, 4021 Linz (AT)
(72) Erfinder: Schwab, Helmut, 8043 Graz (AT); Glieder, Anton, 8200 Gleisdorf (AT); Kratky, Christoph, 8043 Graz (AT); Dreveny, Ingrid, 8010 Graz (AT); Pöchlauer, Peter, 4040 Linz (AT); Skranc, Wolfgang, 1220 Wien (AT); Mayrhofer, Herbert, 4210 Engerwitzdorf (AT); Wirth, Irma, 4470 Enns (AT); Neuhofer, Rudolf, 4210 Mittertreffling (AT); Bona, Rodolfo, 8055 Graz (AT)
(74) Vertreter: Lindinger, Ingrid

(56) Entgegenhaltungen:
- EP-A- 0 276 375
- WO-A-97/03204
- HU ZIHUA ET AL: "Molecular analysis of (R)-(+)-mandelonitrile lyase microheterogeneity in black cherry." PLANT PHYSIOLOGY (ROCKVILLE), Bd. 119, Nr. 4, April 1999 (1999-04), Seiten 1535-1546, XP002195510 ISSN: 0032-0889 -& DATABASE EMBL 1. Oktober 1998 (1998-10-01) "Prunus serotina (R)-(+)-mandelonitrile lyase isoform MDL5 precursor (MDL5), mRNA, complete cds." Database accession no. AF053886 XP002195848 -& DATABASE EMBL 16. Dezember 1996 (1996-12-16) "Prunus serotina (R)-(+)-mandelonitrile lyase isoform MDL1 precursor gene, complete cds." Database accession no. U78814 XP002195849
- SUELVES MONICA ET AL: "Molecular cloning of the cDNA coding for the (R)-(+)-mandelonitrile lyase of Prunus amygdalus: Temporal and spatial expression patterns in flowers and mature seeds." PLANTA (BERLIN), Bd. 206, Nr. 3, 11. Oktober 1998 (1998-10-11), Seiten 388-393, XP002195511 ISSN: 0032-0935 -& DATABASE EMBL 24. September 1996 (1996-09-24) "P. amygdalus mRNA for mandelonitrile lyase" Database accession no. Y08211 XP002195850
- EFFENBERGER F: "ENZYME-CATALYZED PREPARATION AND SYNTHETIC APPLICATIONS OF OPTICALLY ACTIVE CYANOHYDRINS" CHIMIA, AARAU, CH, Bd. 53, Nr. 12, 1999, Seiten 3-10, XP000986127 ISSN: 0009-4293
- CEREGHINO JOAN LIN ET AL: "Heterologous protein expression in the methylotrophic yeast Pichia pastoris." FEMS MICROBIOLOGY REVIEWS, Bd. 24, Nr. 1, Januar 2000 (2000-01), Seiten 45-66, XP002195512 ISSN: 0168-6445

### SEQUENZPROTOKOLL

<110> DSM Fine Chemicals Austria NFG GmbH & Co KG
<120> Neue Gene, enthaltend eine für Hydroxynitrillyase kodierende DNA-Sequenz
<130> O.Z. 1212
<140>
<140>
   <141>
<160> 24
<170> PatentIn Ver. 2.1
<210> 1
   <211> 35
   <212> DNA
   <213> Künstliche Sequenz
<220>
   <223> Beschreibung der künstlichen Sequenz:Primer
<400> 1
   cggaattcac aatatggaga aatcaacaat gtcag 35
<210> 2
   <211> 33
   <212> DNA
   <213> Künstliche Sequenz
<220>
   <223> Beschreibung der künstlichen Sequenz:Primer
<400> 2
   cggaattctt cacatggact cttgaatatt atg 33
<210> 3
   <211> 64
   <212> DNA
   <213> Künstliche Sequenz
<220>
   <223> Beschreibung der künstlichen Sequenz:Primer
<400> 3
   gaagatctga attccatgga gaaatcaaca atgtcagtta tactatttgt gttgcatctt 60
   cttg 64
<210> 4
   <211> 68
   <212> DNA
   <213> Künstliche Sequenz
<220>
   <223> Beschreibung der künstlichen Sequenz:Primer
<400> 4
   ctatttgtgt tgcatcttct tgttcttcat cttcagtatt cagaggttca ctcgcttgcc 60
   aatacttc 68
<210> 5
   <211> 67
   <212> DNA
   <213> Künstliche Sequenz
<220>
   <223> Beschreibung der künstlichen Sequenz:Primer
<400> 5
   gttcactcgc ttgccaatac ttctgctcat gattttagct acttgaagtt tgtgtacaac 60
   gccactg 67
<210> 6
   <211> 33
   <212> DNA
   <213> Künstliche Sequenz
<220>
   <223> Beschreibung der künstlichen Sequenz:Primer
<400> 6
   gatgtattgg aagagaagag gatcttctct act 33
<210> 7
   <211> 56
   <212> DNA
   <213> Künstliche Sequenz
<220>
   <223> Beschreibung der künstlichen Sequeriz:Primer
<400> 7
   gatcctcttc tcttccaata catcaaattt gtcagctatt ggagtcatat atacgg 56
<210> 8
   <211> 66
   <212> DNA
   <213> Künstliche Sequenz
<220>
   <223> Beschreibung der künstlichen Sequenz:Primer
<400> 8
   caaccggatt gacctttctt gcaggatttg aaggcccaca taccttccta acatcagata 60
   gaagcc 66
<210> 9
   <211> 73
   <212> DNA
   <213> Künstliche Sequenz
<220>
   <223> Beschreibung der künstlichen Sequenz:Primer
<400> 9
   gaagatctgg aattcttcac atggactctt gaatattatg aatagcctcc aaccggattg 60
   acctttcttg cag 73
<210> 10
   <211> 114
   <212> DNA
   <213> Künstliche Sequenz
<220>
   <223> Beschreibung der künstlichen Sequenz:Primer
<400> 10
   tcgaattcga gctcggtacc cggggatcct ctagaaataa ttttgtttaa ctttaagaag 60
   gagatataca tatggagaaa tcaacaatgt cagttatact atttgtgttg catc 114
<210> 11
   <211> 54
   <212> DNA
   <213> Künstliche Sequenz
<220>
   <223> Beschreibung der künstlichen Sequenz:Primer
<400> 11
   cgaattcgcc ctttcgcatg ctcacatgga ctcttgaata ttatgaatag cctc 54
<210> 12
   <211> 72
   <212> DNA
   <213> Künstliche Sequenz
<220>
   <223> Beschreibung der künstlichen Sequenz:Primer
<400> 12
   cacgaattca tcatgcagac tctccttgtg agctcgcttg tggtctccct cgctgcggcc 60
   ctgccacact ac 72
<210> 13
   <211> 75
   <212> DNA
   <213> Künstliche Sequenz
<220>
   <223> Beschreibung der künstlichen Sequenz:Primer
<400> 13
   tgcggccctg ccacactaca tcaggagcaa tggcattgaa gcctacaacg ccactgatac 60
   aagctcggaa ggatc 75
<210> 14
   <211> 51
   <212> DNA
   <213> Künstliche Sequenz
<220>
   <223> Beschreibung der künstlichen Sequenz:Primer
<400> 14
   gaattcgcat gcggccgctc actgcattga cctttcttgc aggatttgaa g 51
<210> 15
   <211> 29
   <212> DNA
   <213> Künstliche Sequenz
<220>
   <223> Beschreibung der künstlichen Sequenz:Primer
<400> 15
   actacgaatt cgaccatgga gaaatcaac 29
<210> 16
   <211> 52
   <212> DNA
   <213> Künstliche Sequenz
<220>
   <223> Beschreibung der künstlichen Sequenz:Primer
<400> 16
   cagaattcgc ccttgtgcat gcatcgatta aagaaccaag gatgctgctg ac 52
<210> 17
   <211> 33
   <212> DNA
   <213> Künstliche Sequenz
<220>
   <223> Beschreibung der künstlichen Sequenz:Primer
<400> 17
   actacgaatt cgaccatgga gaaatcaaca atg 33
<210> 18
   <211> 22
   <212> DNA
   <213> Künstliche Sequenz
<220>
   <223> Beschreibung der künstlichen Sequenz:Primer
<400> 18
   atgctgctga cttgagggaa tc 22
<210> 19
   <211> 2162
   <212> DNA
   <213> Prunus amygdalus
<400> 19
   ggaattcaca atatggagaa atcaacaatg tcagttatac tatttgtgtt gcatcttctt 60
   gttcttcatc ttcagtattc agaggttcac tcgcttgcca atacttctgc tcatggtaaa 120
   tttccatctt cagtattcat ttaacagcaa aatgtgtaga tttataatta agaaaactga 180
   cacaagtagt gcaagaaaca agctaattta gatgcatgtt gaaaaaaatc tttcatctct 240
   tcacatatat tttgcagatt ttagctactt gaagtttgtg tacaacgcca ctgatacaag 300
   ctcggaagga tcatatgact acattgtaat cggtggagga acatcagggt gtccattggc 360
   agcaacttta tcagaaaaat acaaggtgct tcttctagaa agaggcacta ttgctacaga 420
   atacccgaac acgttgactg cagatgggtt tgcatataat ctgcagcaac aagatgatgg 480
   aaagacgcca gttgaaaggt tcgtgtccga agatggcatt gataatgtgc gagccaggat 540
   cctcggtggc acgaccataa tcaatgcagg cgtctacgcc agagctaaca tttcattcta 600
   tagtcaaaca ggaattgaat gggacctgga tttggtcaat aagacatatg agtgggttga 660
   agacgccatt gtggtcaagc caaataatca atcttggcaa tctgttatag gagagggatt 720
   cttggaggcg ggtattcttc cagacaatgg atttagtttg gatcacgaag caggaactag 780
   actcaccggc tcaacttttg acaataatgg aacgcgacat gcggctgatg aactgcttaa 840
   taaaggagac cctaataact tgctagttgc agttcaggcc tcagtagaga agatcctctt 900
   ctcttccaat acatcaagta tgttgcatca gtgatattta atggtagctc ctagtttgtc 960
   atgctgcact cgaaaattat tattttatca ttttaaaata ctaacagaat agtgtgaagt 1020
   ctcatatttc ccttccatat ttcccaaatt tccataaaca aaacttccca attctccttc 1080
   gtttagtttg acaataatta taagctattc tctaatgcag atttgtcagc tattggagtc 1140
   atatatacgg attctgatgg aaactctcat caggcatttg tacgcggtaa cggagaagtt 1200
   attgttagtg cagggacaat cggaacgcct cagcttctac tacttagtgg cgttggacca 1260
   gagtcttacc tatcttctct caacatcaca gttgttcagc cgaatcctta tgttgggcag 1320
   tttgtgtatg acaatcctcg taatttcatt aatattttgc ccccaaatcc aattgaagcc 1380
   tctgttgtaa ctgttttagg cattagaagt gattattatc aagtttctct gtcaagcttg 1440
   ccattttcca ctccaccctt tagtcttttt cctacaacat cttaccccct cccaaattcg 1500
   acttttgctc atattgttag ccaagttcca ggaccattgt ctcatggttc tgtcacgcta 1560
   aattcatcat ctgacgtgag aatcgctcca aatattaaat tcaattacta ttcaaattcc 1620
   acagaccttg ctaattgtgt tagcggcatg aagaagcttg gtgacttatt aaggacaaag 1680
   gcattagaac catataaagc tcgagatgtg ctgggaattg acggtttcaa ttatttggga 1740
   gtacctttgc cagagaacca aacagatgat gcatccttcg aaacattttg tctagataat 1800
   gtagcttcat actggcatta ccacggtgga agccttgttg ggaaagtgct tgatgacagt 1860
   ttccgtgtta tggggatcaa agcattacgc gttgttgatg cctccacttt cccttacgaa 1920
   ccaaacagcc atcctcaggg cttctatctg atgttaggaa ggtatgtgat gcacacttcc 1980
   aaccactaga gattctcaat attttgttgt tgttgtaatg aactctctgc cgcattgctc 2040
   ttttttatta atccttaaaa ttttgtgttt tgcgcaggta tgtgggcctt caaatcctgc 2100
   aagaaaggtc aatccggttg gaggctattc ataatattca agagtccatg tgaagaattc 2160
   cg                                                                2162
<210> 20
   <211> 559
   <212> PRT
   <213> Prunus amygdalus
<400> 20
   Met Glu Lys Ser Thr Met Ser Val Ile Leu Phe Val Leu His Leu Leu
   1 5 10 15
   Val Leu His Leu Gln Tyr Ser Glu Val His Ser Leu Ala Asn Thr Ser
   20 25 30
   Ala His Asp Phe Ser Tyr Leu Lys Phe Val Tyr Asn Ala Thr Asp Thr
   35 40 45
   Ser Ser Glu Gly Ser Tyr Asp Tyr Ile Val Ile Gly Gly Gly Thr Ser
   50 55 60
   Gly Cys Pro Leu Ala Ala Thr Leu Ser Glu Lys Tyr Lys Val Leu Leu
   65 70 75 80
   Leu Glu Arg Gly Thr Ile Ala Thr Glu Tyr Pro Asn Thr Leu Thr Ala
   85 90 95
   Asp Gly Phe Ala Tyr Asn Leu Gln Gln Gln Asp Asp Gly Lys Thr Pro
   100 105 110
   Val Glu Arg Phe Val Ser Glu Asp Gly Ile Asp Asn Val Arg Ala Arg
   115 120 125
   Ile Leu Gly Gly Thr Thr Ile Ile Asn Ala Gly Val Tyr Ala Arg Ala
   130 135 140
   Asn Ile Ser Phe Tyr Ser Gln Thr Gly Ile Glu Trp Asp Leu Asp Leu
   145 150 155 160
   Val Asn Lys Thr Tyr Glu Trp Val Glu Asp Ala Ile Val Val Lys Pro
   165 170 175
   Asn Asn Gln Ser Trp Gln Ser Val Ile Gly Glu Gly Phe Leu Glu Ala
   180 185 190
   Gly Ile Leu Pro Asp Asn Gly Phe Ser Leu Asp His Glu Ala Gly Thr
   195 200 205
   Arg Leu Thr Gly Ser Thr Phe Asp Asn Asn Gly Thr Arg His Ala Ala
   210 215 220
   Asp Glu Leu Leu Asn Lys Gly Asp Pro Asn Asn Leu Leu Val Ala Val
   225 230 235 240
   Gln Ala Ser Val Glu Lys Ile Leu Phe Ser Ser Asn Thr Ser Asn Leu
   245 250 255
   Ser Ala Ile Gly Val Ile Tyr Thr Asp Ser Asp Gly Asn Ser His Gln
   260 265 270
   Ala Phe Val Arg Gly Asn Gly Glu Val Ile Val Ser Ala Gly Thr Ile
   275 280 285
   Gly Thr Pro Gln Leu Leu Leu Leu Ser Gly Val Gly Pro Glu Ser Tyr
   290 295 300
   Leu Ser Ser Leu Asn Ile Thr Val Val Gln Pro Asn Pro Tyr Val Gly
   305 310 315 320
   Gln Phe Val Tyr Asp Asn Pro Arg Asn Phe Ile Asn Ile Leu Pro Pro
   325 330 335
   Asn Pro Ile Glu Ala Ser Val Val Thr Val Leu Gly Ile Arg Ser Asp
   340 345 350
   Tyr Tyr Gln Val Ser Leu Ser Ser Leu Pro Phe Ser Thr Pro Pro Phe
   355 360 365
   Ser Leu Phe Pro Thr Thr Ser Tyr Pro Leu Pro Asn Ser Thr Phe Ala
   370 375 380
   His Ile Val Ser Gln Val Pro Gly Pro Leu Ser His Gly Ser Val Thr
   385 390 395 400
   Leu Asn Ser Ser Ser Asp Val Arg Ile Ala Pro Asn Ile Lys Phe Asn
   405 410 415
   Tyr Tyr Ser Asn Ser Thr Asp Leu Ala Asn Cys Val Ser Gly Met Lys
   420 425 430
   Lys Leu Gly Asp Leu Leu Arg Thr Lys Ala Leu Glu Pro Tyr Lys Ala
   435 440 445
   Arg Asp Val Leu Gly Ile Asp Gly Phe Asn Tyr Leu Gly Val Pro Leu
   450 455 460
   Pro Glu Asn Gln Thr Asp Asp Ala Ser Phe Glu Thr Phe Cys Leu Asp
   465 470 475 480
   Asn Val Ala Ser Tyr Trp His Tyr His Gly Gly Ser Leu Val Gly Lys
   485 490 495
   Val Leu Asp Asp Ser Phe Arg Val Met Gly Ile Lys Ala Leu Arg Val
   500 505 510
   Val Asp Ala Ser Thr Phe Pro Tyr Glu Pro Asn Ser His Pro Gln Gly
   515 520 525
   Phe Tyr Leu Met Leu Gly Arg Tyr Val Gly Leu Gln Ile Leu Gln Glu
   530 535 540
   Arg Ser Ile Arg Leu Glu Ala Ile His Asn Ile Gln Glu Ser Met
   545 550 555
<210> 21
   <211> 1632
   <212> DNA
   <213> Künstliche Sequenz
<220>
   <223> Beschreibung der künstlichen Sequenz:PamHNL5xGOX
<400> 21
   gaattcatca tgcagactct ccttgtgagc tcgcttgtgg tctccctcgc tgcggccctg 60
   ccacactaca tcaggagcaa tggcattgaa gcctacaacg ccactgatac aagctcggaa 120
   ggatcatatg actacattgt aatcggtgga ggaacatcag ggtgtccatt ggcagcaact 180
   ttatcagaaa aatacaaggt gcttcttcta gaaagaggca ctattgctac agaatacccg 240
   aacacgttga ctgcagatgg gtttgcatat aatctgcagc aacaagatga tggaaagacg 300
   ccagttgaaa ggttcgtgtc cgaagatggc attgataatg tgcgagccag gatcctcggt 360
   ggcacgacca taatcaatgc aggcgtctac gccagagcta acatttcatt ctatagtcaa 420
   acaggaattg aatgggacct ggatttggtc aataagacat atgagtgggt tgaagacgcc 480
   attgtggtca agccaaataa tcaatcttgg caatctgtta taggagaggg attcttggag 540
   gcgggtattc ttccagacaa tggatttagt ttggatcacg aagcaggaac tagactcacc 600
   ggctcaactt ttgacaataa tggaacgcga catgcggctg atgaactgct taataaagga 660
   gaccctaata acttgctagt tgcagttcag gcctcagtag agaagatcct cttctcttcc 720
   aatacatcaa atttgtcagc tattggagtc atatatacgg attctgatgg aaactctcat 780
   caggcatttg tacgcggtaa cggagaagtt attgttagtg cagggacaat cggaacgcct 840
   cagcttctac tacttagtgg cgttggacca gagtcttacc tatcttctct caacatcaca 900
   gttgttcagc cgaatcctta tgttgggcag tttgtgtatg acaatcctcg taatttcatt 960
   aatattttgc ccccaaatcc aattgaagcc tctgttgtaa ctgttttagg cattagaagt 1020
   gattattatc aagtttctct gtcaagcttg ccattttcca ctccaccctt tagtcttttt 1080
   cctacaacat cttaccccct cccaaattcg acttttgctc atattgttag ccaagttcca 1140
   ggaccattgt ctcatggttc tgtcacgcta aattcatcat ctgacgtgag aatcgctcca 1200
   aatattaaat tcaattacta ttcaaattcc acagaccttg ctaattgtgt tagcggcatg 1260
   aagaagcttg gtgacttatt aaggacaaag gcattagaac catataaagc tcgagatgtg 1320
   ctgggaattg acggtttcaa ttatttggga gtacctttgc cagagaacca aacagatgat 1380
   gcatccttcg aaacattttg tctagataat gtagcttcat actggcatta ccacggtgga 1440
   agccttgttg ggaaagtgct tgatgacagt ttccgtgtta tggggatcaa agcattacgc 1500
   gttgttgatg cctccacttt cccttacgaa ccaaacagcc atcctcaggg cttctatctg 1560
   atgttaggaa ggtatgtggg ccttcaaatc ctgcaagaaa ggtcaatgca gtgagcggcc 1620
   gcatgcgaat tc                                                    1632
<210> 22
   <211> 534
   <212> PRT
   <213> Künstliche Sequenz
<220>
   <223> Beschreibung der künstlichen Sequenz:PamHNL5xGOX
<400> 22
   Met Gln Thr Leu Leu Val Ser Ser Leu Val Val Ser Leu Ala Ala Ala
   1               5                   10                  15
   Leu Pro His Tyr Ile Arg Ser Asn Gly Ile Glu Ala Tyr Asn Ala Thr
               20                  25                  30
   Asp Thr Ser Ser Glu Gly Ser Tyr Asp Tyr Ile Val Ile Gly Gly Gly
   35 40 45
   Thr Ser Gly Cys Pro Leu Ala Ala Thr Leu Ser Glu Lys Tyr Lys Val
   50 55 60
   Leu Leu Leu Glu Arg Gly Thr Ile Ala Thr Glu Tyr Pro Asn Thr Leu
   65 70 75 80
   Thr Ala Asp Gly Phe Ala Tyr Asn Leu Gln Gln Gln Asp Asp Gly Lys
   85 90 95
   Thr Pro Val Glu Arg Phe Val Ser Glu Asp Gly Ile Asp Asn Val Arg
   100 105 110
   Ala Arg Ile Leu Gly Gly Thr Thr Ile Ile Asn Ala Gly Val Tyr Ala
   115 120 125
   Arg Ala Asn Ile Ser Phe Tyr Ser Gln Thr Gly Ile Glu Trp Asp Leu
   130 135 140
   Asp Leu Val Asn Lys Thr Tyr Glu Trp Val Glu Asp Ala Ile Val Val
   145 150 155 160
   Lys Pro Asn Asn Gln Ser Trp Gln Ser Val Ile Gly Glu Gly Phe Leu
   165 170 175
   Glu Ala Gly Ile Leu Pro Asp Asn Gly Phe Ser Leu Asp His Glu Ala
   180 185 190
   Gly Thr Arg Leu Thr Gly Ser Thr Phe Asp Asn Asn Gly Thr Arg His
   195 200 205
   Ala Ala Asp Glu Leu Leu Asn Lys Gly Asp Pro Asn Asn Leu Leu Val
   210 215 220
   Ala Val Gln Ala Ser Val Glu Lys Ile Leu Phe Ser Ser Asn Thr Ser
   225 230 235 240
   Asn Leu Ser Ala Ile Gly Val Ile Tyr Thr Asp Ser Asp Gly Asn Ser
   245 250 255
   His Gln Ala Phe Val Arg Gly Asn Gly Glu Val Ile Val Ser Ala Gly
   260 265 270
   Thr Ile Gly Thr Pro Gln Leu Leu Leu Leu Ser Gly Val Gly Pro Glu
   275 280 285
   Ser Tyr Leu Ser Ser Leu Asn Ile Thr Val Val Gln Pro Asn Pro Tyr
   290 295 300
   Val Gly Gln Phe Val Tyr Asp Asn Pro Arg Asn Phe Ile Asn Ile Leu
   305 310 315 320
   Pro Pro Asn Pro Ile Glu Ala Ser Val Val Thr Val Leu Gly Ile Arg
   325 330 335
   Ser Asp Tyr Tyr Gln Val Ser Leu Ser Ser Leu Pro Phe Ser Thr Pro
   340 345 350
   Pro Phe Ser Leu Phe Pro Thr Thr Ser Tyr Pro Leu Pro Asn Ser Thr
   355 360 365
   Phe Ala His Ile Val Ser Gln Val Pro Gly Pro Leu Ser His Gly Ser
   370 375 380
   Val Thr Leu Asn Ser Ser Ser Asp Val Arg Ile Ala Pro Asn Ile Lys
   385 390 395 400
   Phe Asn Tyr Tyr Ser Asn Ser Thr Asp Leu Ala Asn Cys Val Ser Gly
   405 410 415
   Met Lys Lys Leu Gly Asp Leu Leu Arg Thr Lys Ala Leu Glu Pro Tyr
   420 425 430
   Lys Ala Arg Asp Val Leu Gly Ile Asp Gly Phe Asn Tyr Leu Gly Val
   435 440 445
   Pro Leu Pro Glu Asn Gln Thr Asp Asp Ala Ser Phe Glu Thr Phe Cys
   450 455 460
   Leu Asp Asn Val Ala Ser Tyr Trp His Tyr His Gly Gly Ser Leu Val
   465 470 475 480
   Gly Lys Val Leu Asp Asp Ser Phe Arg Val Met Gly Ile Lys Ala Leu
   485 490 495
   Arg Val Val Asp Ala Ser Thr Phe Pro Tyr Glu Pro Asn Ser His Pro
   500 505 510
   Gln Gly Phe Tyr Leu Met Leu Gly Arg Tyr Val Gly Leu Gln Ile Leu
   515 520 525
   Gln Glu Arg Ser Met Gln
   530
<210> 23
   <211> 2087
   <212> DNA
   <213> Prunus amygdalus
<400> 23
   atggagaaat caacaatgtc agctatactg ttggtgttat acatttttgt cctccatctt 60
   caatattctg aggtccactc gcttgccacg acttctgatc atggtaaatc acttcaaccg 120
   taattcaaaa caccaaaaag gcaatcaaaa agaaaacgga aaaaagtgta agaaaagcag 180
   atatagacgc ctgcatagat gcatgtgcta tatactttta aaaactcttc gtctcttgag 240
   attttgcaga ttttagctac ctgagctttg catacgacgc cactgatcta gagttggaag 300
   gatcatatga ctacgttata gttggcggag gaacatcagg gtgtccattg gcagcaactt 360
   tatcagaaaa atacaaggtg ctcgttctcg aaaggggcag tcttccgaca gcatatccca 420
   acgtcttgac tgcagatggg tttgtatata atctccagca agaagatgat ggaaagacac 480
   cggtcgaaag gttcgtgtcc gaagatggta ttgataatgt acggggcagg gtgctcggtg 540
   gcacaagcat tatcaatgcc ggtgtctacg ccagagctaa cacctcaatc tatagtgcat 600
   caggagttga ttgggacatg gatttggtta atcagacata tgagtgggtt gaagacacta 660
   ttgtgtacaa gccaaattct caatcttggc agtctgttac aaaaactgca ttcttggagg 720
   ctggtgttca tccaaaccat ggatttagtt tagatcatga agaaggaact agaattaccg 780
   gctcaacttt tgacaacaag ggaacgagac atgcagctga tgaacttctt aataaaggaa 840
   actctaacaa cttgcgagtt ggagttcatg cctcagtaga gaagatcatc ttctccaatg 900
   caccaggtat gttgcatcat gcactccaaa attaatattt tgtcatttta aaacactagc 960
   aggagccaag gtctggaagt acgaataaaa tttcattatt ttccttggat ttgtttgata 1020
   atgattataa gcttttctgt aatgtaggtt tgacagctac aggagtcata tatagggatt 1080
   ctaatggaac gcctcaccaa gcatttgtac gcagtaaggg agaagttatc gtgagtgcag 1140
   ggacaattgg gacccctcaa cttctactac ttagcggtgt tgggccagag tcttacctat 1200
   catctctaaa tattccagtt gttctttccc atccttacgt cggacagttt ctgcatgaca 1260
   atcctcgtaa tttcattaac attttgcccc caaatccaat tgaacccaca attgtaactg 1320
   ytctaggcat ttcaaacgat ttctaccaat gttctttctc gagcttgcca tttacaactc 1380
   cacccttcgg ttttttccct agtgcatctt atcccctgcc aaattcgact tttgctcact 1440
   ttgctagcaa agtggcagga cctttatcat atggttctct cacactgaaa tcatcctcca 1500
   atgtgagagt cagtccaaat gtcaaattta attactattc aaatctgaca gatctttctc 1560
   attgtgttag cggcatgaag aagattggtg aactcttgag cacagacgca ttaaaaccat 1620
   ataaagttga agatttgccg ggtgtagaag gttttaatat tttgggaatc cctttgccaa 1680
   aggaccaaac agatgatgca gccttcgaaa cattttgccg agaatcagta gcctcatatt 1740
   ggcactacca cggtggatgc cttgttggaa aggtgcttga tggtgatttc cgtgttacag 1800
   ggatcaatgc attacgcgtt gttgatggct caacattccc ttacacacca gcgagccacc 1860
   ctcagggctt ctatctgatg ttagggaggt atgttacaaa ttctcaataa ttatttggtt 1920
   gagtggcttg tttgtaatga actctatgcc atatttctct ttctcatcct ttccattttt 1980
   gtgccatggg caggtatgtg ggcattaaaa ttctgcaaga aagatcagct tcagatctaa 2040
   aaatcttgga ttccctcaag tcagcagcat ccttggttct ttaaact            2087
<210> 24
   <211> 563
   <212> PRT
   <213> Prunus amygdalus
<400> 24
   Met Glu Lys Ser Thr Met Ser Ala Ile Leu Leu Val Leu Tyr Ile Phe
   1               5                   10                  15
   Val Leu His Leu Gln Tyr Ser Glu Val His Ser Leu Ala Thr Thr Ser
               20                  25                  30
   Asp His Asp Phe Ser Tyr Leu Ser Phe Ala Tyr Asp Ala Thr Asp Leu
           35                  40                  45
   Glu Leu Glu Gly Ser Tyr Asp Tyr Val Ile Val Gly Gly Gly Thr Ser
       50                  55                  60
   Gly Cys Pro Leu Ala Ala Thr Leu Ser Glu Lys Tyr Lys Val Leu Val
   65                  70                  75                  80
   Leu Glu Arg Gly Ser Leu Pro Thr Ala Tyr Pro Asn Val Leu Thr Ala
                   85                  90                  95
   Asp Gly Phe Val Tyr Asn Leu Gln Gln Glu Asp Asp Gly Lys Thr Pro
               100                 105                 110
   Val Glu Arg Phe Val Ser Glu Asp Gly Ile Asp Asn Val Arg Gly Arg
           115                 120                 125
   Val Leu Gly Gly Thr Ser Ile Ile Asn Ala Gly Val Tyr Ala Arg Ala
       130                 135                 140
   Asn Thr Ser Ile Tyr Ser Ala Ser Gly Val Asp Trp Asp Met Asp Leu
   145                 150                 155                 160
   Val Asn Gln Thr Tyr Glu Trp Val Glu Asp Thr Ile Val Tyr Lys Pro
                   165                 170                 175
   Asn Ser Gln Ser Trp Gln Ser Val Thr Lys Thr Ala Phe Leu Glu Ala
               180                 185                 190
   Gly Val His Pro Asn His Gly Phe Ser Leu Asp His Glu Glu Gly Thr
           195                 200                 205
   Arg Ile Thr Gly Ser Thr Phe Asp Asn Lys Gly Thr Arg His Ala Ala
       210                 215                 220
   Asp Glu Leu Leu Asn Lys Gly Asn Ser Asn Asn Leu Arg Val Gly Val
   225                 230                 235                 240
   His Ala Ser Val Glu Lys Ile Ile Phe Ser Asn Ala Pro Gly Leu Thr
                   245                 250                 255
   Ala Thr Gly Val Ile Tyr Arg Asp Ser Asn Gly Thr Pro His Gln Ala
   260 265 270
   Phe Val Arg Ser Lys Gly Glu Val Ile Val Ser Ala Gly Thr Ile Gly
   275 280 285
   Thr Pro Gln Leu Leu Leu Leu Ser Gly Val Gly Pro Glu Ser Tyr Leu
   290 295 300
   Ser Ser Leu Asn Ile Pro Val Val Leu Ser His Pro Tyr Val Gly Gln
   305 310 315 320
   Phe Leu His Asp Asn Pro Arg Asn Phe Ile Asn Ile Leu Pro Pro Asn
   325 330 335
   Pro Ile Glu Pro Thr Ile Val Thr Val Leu Gly Ile Ser Asn Asp Phe
   340 345 350
   Tyr Gln Cys Ser Phe Ser Ser Leu Pro Phe Thr Thr Pro Pro Phe Gly
   355 360 365
   Phe Phe Pro Ser Ala Ser Tyr Pro Leu Pro Asn Ser Thr Phe Ala His
   370 375 380
   Phe Ala Ser Lys Val Ala Gly Pro Leu Ser Tyr Gly Ser Leu Thr Leu
   385 390 395 400
   Lys Ser Ser Ser Asn Val Arg Val Ser Pro Asn Val Lys Phe Asn Tyr
   405 410 415
   Tyr Ser Asn Leu Thr Asp Leu Ser His Cys Val Ser Gly Met Lys Lys
   420 425 430
   Ile Gly Glu Leu Leu Ser Thr Asp Ala Leu Lys Pro Tyr Lys Val Glu
   435 440 445
   Asp Leu Pro Gly Val Glu Gly Phe Asn Ile Leu Gly Ile Pro Leu Pro
   450 455 460
   Lys Asp Gln Thr Asp Asp Ala Ala Phe Glu Thr Phe Cys Arg Glu Ser
   465 470 475 480
   Val Ala Ser Tyr Trp His Tyr His Gly Gly Cys Leu Val Gly Lys Val
   485 490 495
   Leu Asp Gly Asp Phe Arg Val Thr Gly Ile Asn Ala Leu Arg Val Val
   500 505 510
   Asp Gly Ser Thr Phe Pro Tyr Thr Pro Ala Ser His Pro Gln Gly Phe
   515 520 525
   Tyr Leu Met Leu Gly Arg Tyr Val Gly Ile Lys Ile Leu Gln Glu Arg
   530 535 540
   Ser Ala Ser Asp Leu Lys Ile Leu Asp Ser Leu Lys Ser Ala Ala Ser
   545 550 555 560
   Leu Val Leu

## Beschreibung

Biokatalytische Verfahren haben für die chemische industrie eine hohe Bedeutung erlangt. Die Durchführung chemischer Reaktionen unter Zuhilfenahme biologischer Katalysatoren ist dabei vor allem in solchen Anwendungsgebieten von Interesse, in denen die oft vorhandene Eigenschaft von Enzymen, bei chemischen Reaktionen mit chiralen oder prochiralen Komponenten eines der beiden Enantiomeren bevorzugt umzusetzen oder zu bilden, genutzt werden kann.
Wesentliche Voraussetzungen für die Nutzung dieser günstigen Eigenschaften von Enzymen sind ihre Verfügbarkeit in technisch benötigten Mengen und eine ausreichend hohe Reaktivität, sowie Stabilität unter den realen Bedingungen eines technischen Verfahrens.
Eine besonders interessante Klasse von chiralen chemischen Verbindungen sind Cyanhydrine. Cyanhydrine sind beispielsweise bei der Synthese von α-Hydroxysäuren, α-Hydroxyketonen, β-Aminoalkoholen, die zur Gewinnung biologisch wirksamer Stoffe, z.B. pharmazeutischer Wirkstoffe, Vitamine oder pyrethroider Verbindungen Verwendung finden, von Bedeutung.
Die Herstellung dieser Cyanhydrine erfolgt durch Addition von Blausäure an die Carbonylgruppe eines Ketons oder Aldehyds.
Die technische Herstellung von chiralen Verbindungen, wie etwa (S)-Cyanhydrinen, konnte durch die Erschließung des Enzyms (S)-Hydroxynitril Lyase aus *Hevea brasiliensis* realisiert werden und ist beispielsweise in WO 97/03204, EP 0 951561 und EP 0 927 766 beschrieben.
Es gibt jedoch eine Vielfalt an interessanten chemischen Verbindungen, bei welchen die R-Enantiomeren für technische Anwendungen von Bedeutung sind. Bisher wurden lediglich Verfahren zur Herstellung einer Reihe von Produkten beschrieben, die nur im Labormaßstab eingesetzt werden können (z.B.: EP 0 276 375, EP 0 326 063, EP 0 547 655). Dabei wurden vorwiegend Enzympräparationen eingesetzt, die aus Pflanzen der Familie Rosaceae, beispielsweise aus den Kernen von Mandeln (*Prunus amygdalus*) gewonnen wurden.
In letzter Zeit gewannen *Prunus spezies* immer mehr an Bedeutung, sodass versucht wurde, diese genauer zu erforschen.

Aus der Fachliteratur, beispielsweise aus Plant Physiology, April 1999, Vol 119, pp. 1535-1546, ist bekannt, dass in *Prunus spezies* mehrere R-HNL Isoenzyme vorkommen können. Diese Isoenzyme sind in verschiedenen Geweben der Pflanze unterschiedlich stark exprimiert. Bei der zu *Prunus amygdalus* nahe verwandten Pflanze *Prunus serotina* konnten bislang 5 verschiedene Isoenzyme identifiziert und deren Gene sequenziert werden. Von *Prunus amygdalus* ist bislang nur ein lsoenzym in Planta (1998) 206: 388-393 beschrieben worden, nämlich eines das in der Blütenknospe am stärksten exprimiert ist. Ein Gen für dieses R-HNL Isoenzyme wurde bereits isoliert und die cDNA sequenziert.
Es ist jedoch noch kein Bericht einer erfolgreichen (funktionellen) heterologen Expression eines solchen Gens in der Fachliteratur oder Patentliteratur beschrieben.

Auch technische Anwendungen im größeren Maßstab sind bislang nicht realisiert worden. Die wesentliche Ursache dafür ist, dass Enzympräparationen aus Mandelkernen mit Hydroxynitrillyase-Aktivität bisher nicht in ausreichenden Mengen und zu vertretbaren Kosten verfügbar waren.

Ziel dieser Erfindung war es daher eine Basis zu schaffen, die eine R-Hydroxynitrillyase in für technische Anwendungen benötigten Mengen bereitstellen kann.

Zur Erreichung dieses Zieles wurde ein Weg gesucht, ein der R-HNL Präparation von *Prunus amygdalus* entsprechendes Enzym durch gentechnische Strategien mit Hilfe eines entsprechenden rekombinanten Mikroorganismenstammes zu produzieren. Ein solches rekombinates Enzym mit R-HNL Aktivität sollte auf diese Art in ausreichender Menge technisch verfügbar gemacht werden.

Aus den in obiger Literatur beschriebenen DNA Sequenzen kann abgeleitet werden, dass bei den Genen der verschiedenen lsoenzyme bestimmte Bereiche hoch konserviert sind. Das wird erfindungsgemäß als Grundlage herangezogen, Primer für die PCR-Amplifikation von *P.amygdalus* R-HNL Genen zu generieren. Mit Hilfe solcher Primer können dann mit aus beispielsweise Mandelkernen (*P. amygdalus*) isolierter DNA als Template (Vorlage) mittels PCR DNA-Stücke amplifiziert werden, die bei Analyse durch Agarose-Gelelektrophorese konkrete spezifische Banden zeigen. Erfindungsgemäß waren dabei Banden zu finden, die der Größe von mdl Genen (Planta (1998) 206: 388-393) entsprachen. Anschließend werden entsprechende Primer für alle bei *Prunus serotina* bekannten Isoenzyme generiert und entsprechende PCR Produkte gewonnen. DNA aus diesem Bereich wird aus entsprechenden präparativen Agarosegelen isoliert und in Standardvektoren für die Klonierung von PCR-generierten Fragmenten in *Escherichia coli* einkloniert.
Die Sequenzanalyse von einer Serie ausgewählter Klone ergab, dass Klone mit Homologien zu den jeweiligen bereits bekannten R-HNL Genen von Prunusarten vorhanden sind, obwohl sich die Sequenzen der so erhaltenen Klone bzw. Gene in mehreren Sequenzpositionen von den bereits bekannten bzw. publizierten Sequenzen unterscheiden, wodurch wichtige funktionelle Unterschiede festgelegt werden.
Es wurde daher unerwarteterweise eine neue Variante der HNL Gene gefunden, obwohl Primerkombinationen verwendet wurden, bei der die bereits bekannte Sequenz einer aus Blütenmaterial von *Prunus amygdalus* gewonnenen cDNA herangezogen wurde.
Das neue Gen wurde sequenziert und die genomische DNA-Sequenz ermittelt.

Gegenstand der voliegenden Erfindung ist demnach ein neues Gen, welches die Nukleotidsequenz SEQ ID NO: 19 von Nukleotid 13 bis Nukleotid 2151 durchgehend oder diese Sequenz abzüglich der Intron-Bereiche von den Nukleotiden 116 bis 257, 918 bis 1120 und 1962 bis 2077 umfasst.

So können genspezifische PCR Primer basierend auf der Sequenzhomologie des MDL1 Gens von *Prunus amygdalus* und des *mdl5* Gens von *Prunus serotina* hergestellt werden, worauf nach Amplifikation und Klonierung das neue Gen, das *HNL5* Gen, erhalten wird.

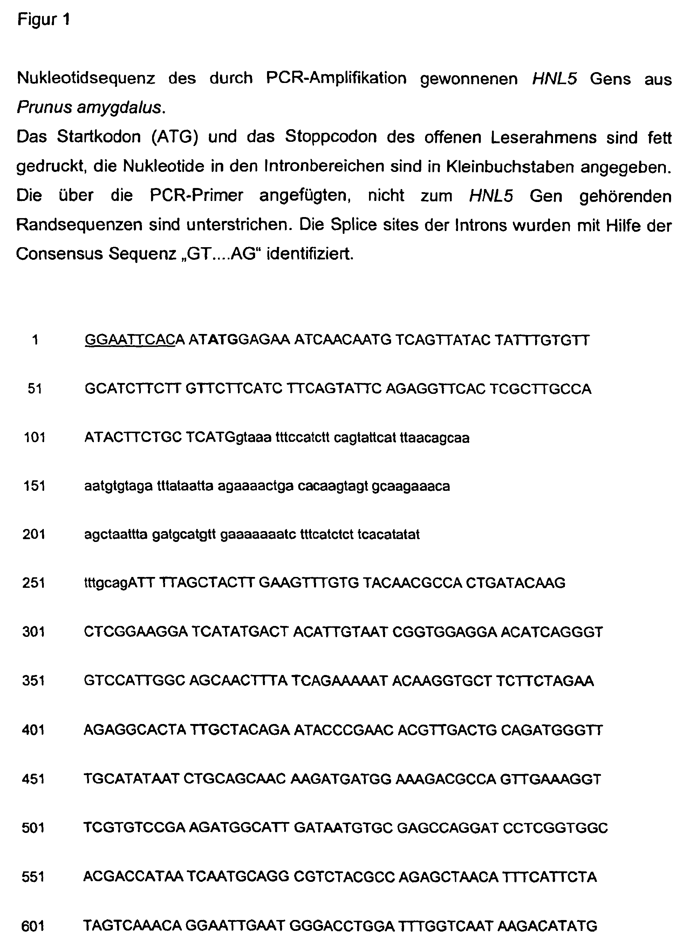

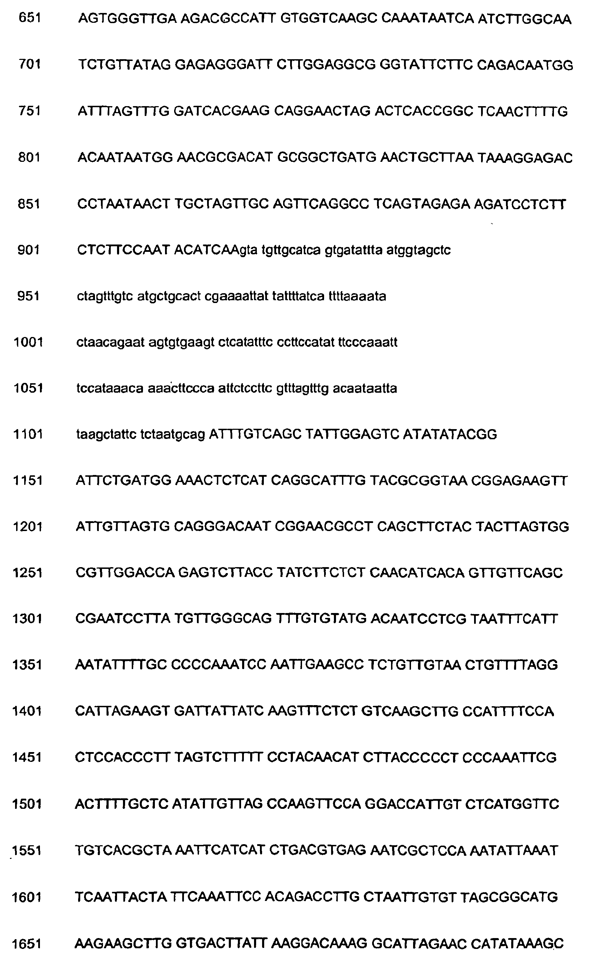

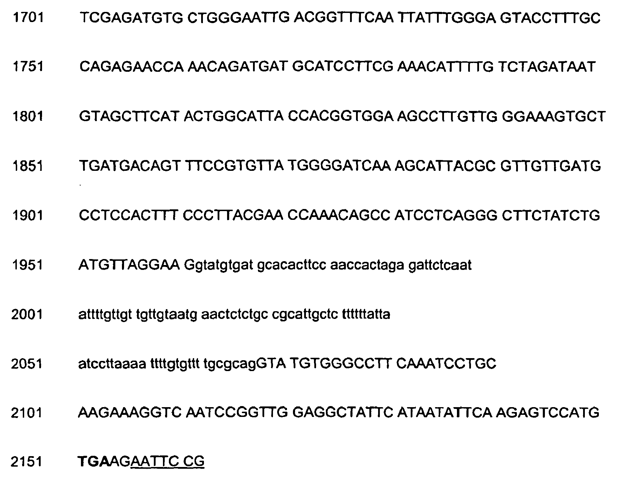

Beispielsweise weist das durch PCR-Amplifikation gewonnene *HNL5* Gen aus *Prunus amygdalus* die in Figur 1 abgebildete Nukleotidsequenz (SEQ ID NO:19) auf.

Das neue *HNL5* Gen unterscheidet sich von der publizierten Sequenz des MDL1 Gens von *Prunus amygdalus* in 7 Basenpaaren.

Die genomischen Klone bzw. deren genomische DNA stellen die Basis für die Gewinnung von Enzympräparationen durch heterologe Expression, beispielsweise durch induzierbare oder konstitutive Expression, in verschiedenen Wirtszellen dar.

Weiters ist aus der Sequenzanalyse der genomischen DNA des neuen, erfindungsgemäßen Gens ersichtlich, dass es sich bei dem von dem neuen Gen kodierten Protein um ein Protein handelt, das über eine Signalsequenz bzw. ein pflanzliches Signalpeptid verfügt, wodurch auch eine sekretorische Expression von heterologen Proteinen in geeigneten Wirtszellen ermöglicht wird.
Dabei werden spezielle Expressionsvektoren verwendet, mit denen das von einem der einklonierten neuen Gene codierte Protein als Fusionsprotein mit einem Signalpeptid exprimiert wird.

Die vorliegende Erfindung betrifft demnach weiters rekombinante Proteine, herstellbar durch heterologe Expression der DNA-Sequenz des *HNL5* Gens aus *Prunus amygdalus* in geeigneten Wirtszellen.
Geeignete Wirtszellen sind dabei beispielsweise Mikroorganismen. Bevorzugt sind eukaryotische Mikroorganismen, besonders bevorzugt Pilze. Beispiele dafür sind *Saccharomyces cerevisiae* oder *Pichia pastoris.*

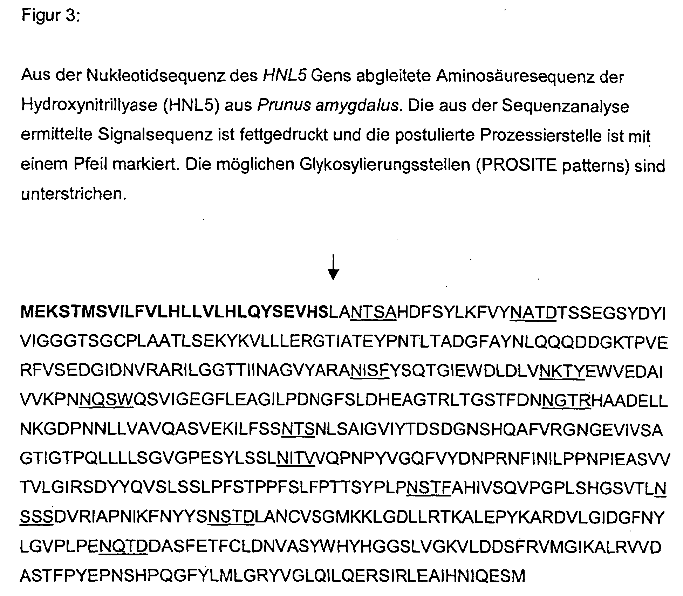

Die aus der Nukleotidsequenz des *HNL5* Gens abgeleitete Aminosäuresequenz der Hydroxynitrillyase HNL5 aus *Prunus amygdalus* ist in Figur 3 (SEQ ID NO: 20) dargestellt.

Um rekombinante Proteine mit Hydroxynitrillyase Aktivität zu erhalten wird das entsprechende *HNL5* Gen, z.B. in einen Expressionsvektor für *Pichia pastoris* oder für *Saccharomyces cerevisiae* eingebaut.
Zuvor kann die genomische DNA mittels PCR gespleisst werden. Bevorzugt wird ein Basisfragment zur Konstruktion von Expressionsplasmiden für die heterologe Expression des entsprechenden Gens in Bakterien und Eukaryonten hergestellt. Dazu wird ein Plasmid konstruiert, aus dem ein für ein erfindungsgemäßes Gen kodierendes DNA Fragment für den Einbau in verschiedene Expressionsvektoren durch Ausschneiden mit Restriktionsendonukleasen gewonnen werden kann.

Mit dem erfindungsgemäßen Gen kann somit durch Expression, beispielsweise mit einem induzierbaren Promotersystem oder einem konstitutiv exprimierenden Promotorsystem, in einem heterologen Wirt eine funktionelle HNL produziert werden.
Aus einer großen Anzahl von Transformanten können somit rekombinante Stämme von beispielsweise *Pichia pastoris* gefunden werden, die rekombinantes Protein mit R-HNL Aktivität überexprimieren. Das Protein mit R-HNL Aktivität ist bei diesen Stämmen nach Induktion der Expression zum überwiegenden Teil im Kulturüberstand zu finden.

Somit konnte erstmals ein rekombinantes Protein in für technische Anwendungen nutzbaren Mengen hergestellt werden, das eine zu in Mandelkernen (Kerne von *Prunus amygdalus*) aufzufindende vergleichbare R-HNL Aktivität aufweist.

Unerwarteterweise konnte festgestellt werden, dass das rekombinante Protein mit R-HNL Aktivität, das sich von dem erfindungsgemäßen *HNL5* Gen ableitet und mit Pam-HNL5 bezeichnet wird, im Vergleich zum aus Mandelkernen isolierten Enzympräparat wesentlich bessere Eigenschaften als Biokatalysator in Reaktionsansätzen zur Herstellung von Cyanhydrinen aufweist und somit besonders vorteilhaft für die biokatalytische Synthese von Cyanhydrinen verwendet werden kann.

Die Sequenz des erfindungsgemäßen rekombinanten Proteins kann weiters auch noch am C-terminalen Ende verkürzt werden, bzw. kann die Sequenz im N- und C-terminalen Bereich durch solche der Glukoseoxidase von *Aspergillus niger* ausgetauscht werden.

Das rekombinante Protein zeichnet sich insbesondere auch dadurch aus, dass es eine wirtsspezifische Glykosilierung aufweiset, wodurch das rekombinante Protein wesentlich stabiler ist als die nativen Proteine.
Ein besonderer Vorteil des erfindungsgemäßen rekombinanten Proteins ergibt sich durch die wesentlich höhere Stabilität, durch die wesentlich weniger Enzymmenge im Vergleich zu nativem Enzym benötigt wird um hohe Enantiomerenreinheit zu erzielen. So zeigten Vergleichsversuche, dass bei Verwendung des erfindungsgemäßen rekombinanten Proteins lediglich ein Zehntel der benötigten Menge an nativem Protein benötigt wird, um vergleichbare Enantiomerenreinheiten (ee-Werte) zu erzielen.

Ein weiterer Gegenstand der Erfindung ist demnach die Verwendung des erfindungsgemäßen rekombinanten Proteins (HNL) zur Herstellung von (S)- oder (R)-Cyanhydrinen.

Als Ausgangsmaterialien zur Herstellung der (S)- oder (R)-Cyanhydrinen werden ein Aldehyd oder ein Keton als Substrat, ein Cyanidgruppendonor und das erfindungsgemäßes rekombinantes Protein eingesetzt.

Unter Aldehyden sind dabei aliphatische, aromatische oder heteroaromatische Aldehyde zu verstehen. Unter aliphatischen Aldehyden sind dabei gesättigte oder ungesättigte, aliphatische, geradkettige, verzweigte oder cyclische Aldehyde zu verstehen. Bevorzugte aliphatische Aldehyde sind geradkettige Aldehyde mit insbesondere 2 bis 30 C-Atomen, bevorzugt von 2 bis 18 C-Atomen, die gesättigt oder ein- oder mehrfach ungesättigt sind. Der Aldehyd kann dabei sowohl C-C-Doppelbindungen als auch C-C-Dreifachbindungen aufweisen. Die aliphatischen, aromatischen oder heteroaromatischen Aldehyde können weiters unsubstituiert oder durch unter den Reaktionsbedingungen inerte Gruppen, beispielsweise durch gegebenenfalls substituierte Aryl- oder Heteroarylgruppen, wie Phenyl-, Phenoxy- oder Indolylgruppen, durch Halogen-, Hydroxy-, Hydroxy-C₁-C₅-Alkyl, C₁-C₅-Alkoxy-, C₁-C₅-Alkylthio-, Ether-, Alkohol-, Carbonsäureester-, Nitro- oder Azidogruppen substituiert sein.
Beispiele für aromatische oder heteroaromatische Aldehyde sind Benzaldezyd bzw. verschieden substituierte Benzaldehyde wie etwa 3,4-Difluorbenzaldehyd, 3-Phenoxybenzaldehyd, 4-Fluor-3-phenoxybenzaldehyd, Hydroxybenzaldehyd, Methoxybenzaldehyd, weiters Furfural, Methylfurfural, Anthracen-9-carbaldehyd, Furan-3-carbaldehyd, Indol-3-carbaldehyd, Naphthalin-1-carbaldehyd, Phthaldialdehyd, Pyrazol-3-carbaldehyd, Pyrrol-2-carbaldehyd, Thiophen-2-carbaldehyd, lsophthalaldehyd oder Pyridinaldehyde, Thienylaldehyde u.s.w..
Ketone sind aliphatische, aromatische oder heteroaromatische Ketone, bei denen das Carbonylkohlenstoffatom ungleich substituiert ist. Unter aliphatischen Ketonen sind gesättigte oder ungesättigte, geradkettige, verzweigte oder cyclische Ketone zu verstehen. Die Ketone können gesättigt oder ein- oder mehrfach ungesättigt sein. Sie können unsubstituiert, oder durch unter Reaktionsbedingungen inerte Gruppen, beispielsweise durch gegebenenfalls substituierte Aryl- oder Heteroarylgruppen wie Phenyl- oder Inolylgruppen, durch Halogen-, Ether-, Alkohol-, Carbonsäureester-, Nitro- oder Azidogruppen substituiert sein.
Beispiele für aromatische oder heteroaromatische Ketone sind Acetophenon, Indolylaceton u.s.w..

Aldehyde und Ketone, die sich erfindungsgemäß eignen, sind bekannt oder wie üblich herstellbar.

Die Substrate werden in Anwesenheit der erfindungsgemäßen HNL mit einem Cyanidgruppendonor umgesetzt.

Als Cyanidgruppendonor kommen Blausäure, Alkali-Cyanide oder ein Cyanhydrin der allgemeinen Formel I

R₁R₂C(OH)(CN),

in Betracht. In der Formel I bedeuten R₁ und R₂ unabhängig voneinander Wasserstoff oder eine unsubstituierte Kohlenwasserstoffgruppe, oder R₁ und R₂ gemeinsam eine Alkylengruppe mit 4 oder 5 C-Atomen, wobei R₁ und R₂ nicht gleichzeitig Wasserstoff bedeuten. Die Kohlenwasserstoffgruppen sind aliphatische oder aromatische, bevorzugt aliphatische Gruppen. Bevorzugt bedeuten R₁ und R₂ Alkylgruppen mit 1 - 6 C-Atomen, ganz bevorzugt ist der Cyanidgruppendonor Acetoncyanhydrin.

Die Herstellung des Cyanidgruppendonors kann nach bekannten Verfahren erfolgen. Cyanhydrine, insbesonders Acetoncyanhydrin, sind auch käuflich zu erwerben.
Bevorzugt wird Blausäure (HCN), KCN, NaCN, oder Acetoncyanhydrin, besonders bevorzugt Blausäure als Cyanidgruppendonor eingesetzt.

Die Blausäure kann dabei auch erst kurz vor der Reaktion aus einem ihrer Salze wie etwa NaCN oder KCN freigesetzt und in Substanz oder in gelöster Form dem Reaktionsgemisch zugegeben werden.

Die Umsetzung kann im organischen, wäßrigen oder 2-Phasensystem oder in Emulsion durchgeführt werden.
Als wäßriges System wird eine wäßrige, die erfindungsgemäße HNL enthaltende Lösung oder Pufferlösung verwendet. Beispiele dafür sind Na-Citratpuffer, Phosphatpuffer u.s.w.

Als organisches Verdünnungsmittel können mit Wasser nicht oder geringfügig mischbare aliphatische oder aromatische Kohlenwasserstoffe, die gegebenenfalls halogeniert sind, Alkohole, Ether oder Ester oder Gemische davon verwendet werden. Bevorzugt werden Methyl -tert.Butylether (MTBE), Diisopropylether, Dibutylether und Ethylacetat oder deren Gemisch eingesetzt.
Die erfindungsgemäße HNL kann dabei entweder als solche oder immobilisiert in dem organischen Verdünnungsmittel vorliegen, die Umsetzung kann jedoch auch in einem Zweiphasensystem oder in Emulsion mit nicht-immobilisierter HNL erfolgen.

### Beispiel 1:

### Isolierung von genomischer DNA aus Mandelkernen (Kerne von Prunus amygdalus)

Getrocknete Mandelkerne (Farmgold, Chargennummer L4532, Ernte 1999) wurden mit einem Messer fein gehackt und in einer Reibschale mit flüssigem Stickstoff eingefroren und unter flüssigem Stickstoff mit einem Pistill zu einem feinen Pulver aufgerieben. 0,1 Gramm gefrorenes Mandelkernpulver wurden direkt mit 65 °C warmem "Breaking Puffer" (100 mM NaAc; 50 mM EDTA; 500 mM NaCl, eingestellt auf pH 5,5; 1,4% SDS und 20µg / ml RNAse A) versetzt. Nach 15 minütiger Inkubation unter ständigem Schütteln wurden die unlöslichen Zellrückstände durch Zentrifugation (10 min. bei 7000 g) abgetrennt und der Überstand mit einem gleichen Volumen 10M Ammoniumacetat versetzt und anschließend 10 min auf Eis inkubiert. Nach 15 minütiger Zentrifugation bei 10.000g wurde der Überstand 2 × mit Phenol/Chloroform (1/1, Phenol äquilibriert mit 50 mM Tris, pH 8.0) extrahiert. Nach einer weiteren Extraktion mit doppeltem Volumen Chloroform/Isoamylalkohol (24/1) wurde die DNA aus dem Überstand mit einem gleichen Volumen Isopropanol gefällt, abzentrifugiert, das DNA Pellet mit 70% Äthanol gewaschen und luftgetrocknet. Die DNA wurde dann für 20 min bei 68°C in 200 µl Wasser gelöst und durch eine Ethanolfällung (Ausubel et al., 1999) gereinigt. Nach der Zentrifugation wurde das DNA Pellet luftgetrocknet und in 50 µl Wasser gelöst.

### Beispiel 2:

### Amplifikation und Klonierung eines genomischen DNA Abschnittes von Mandel (Prunus amygdalus) DNS mit Homologie zu bekannten mdl Genen von Rosaceae.

Da bekannt war, dass in *Prunus spezies* mehrere Isoenzyme von Hydroxyntrillylasen mit hoher Sequenzhomologie zueinander auftreten können (Hu und Poulton, 1999), wurden genspezifische PCR Primer basierend auf der Sequenzhomologie des *mdl5* Gens von *Prunus serotina* und des *MDL1* Gens von *Prunus amygdalus* (Suelves et al., 1998) hergestellt:

Die Amplifikation erfolgte in einem 50 µl Ansatz mit 1,2 U "Hotstar" *Taq* DNA Polymerase (Qiagen, Hilden, Deutschland), mit 50 ng genomischer Mandel-DNA als "Template", je 200 ng Primer 1 und 2, 5 µl eines dNTP (je 2 mM) Mix, alles in 1x PCR Puffer entsprechend dem Manual des "Hotstar Kit" (Qiagen, Hilden, Deutschland), beginnend mit einem 15 minütigen Denaturierungsschritt bei 95°C, gefolgt von 30 Zyklen (1 min 95°C, 30 sec 64°C, 1 min 72°C) zur Amplifikation und einer abschließenden Inkubation für 5 min bei 72 °C zur Herstellung vollständiger Produkte.
Durch diese PCR wurde ein DNA Fragment in der Grösse von ca. 2,16 kb (festgestellt durch Analyse mittels Agrose-Gelelektrophorese) erhalten. Dieses PCR Produkt wurde mittels "Qiaquick Kit" (Qiagen. Hilden, Deutschland) entsprechend dem beigefügten Manual gereinigt und unter Verwendung des "Dye Deoxy Terminator Cycle Sequencing" Kits (Applied Biosystems Inc., Forster City, CA, USA) nach der Strategie des "Primer walking" ausgehend von den beiden zur PCR eingesetzten Primern sequenziert. Die erhaltene DNA Sequenz des insgesamt 2162 Basenpaare langen PCR Fragments ist in Figur 1 dargestellt.
Ca. 0,5 µg des gereinigten PCR Produktes wurden mit der Restriktionsendonuklease *Eco*RI geschnitten und in den Plasmidvektor pBSSK(-) (Stratagene Cloning Systems, La Jolla, CA, USA) über die *Eco*RI Schnittstelle einkloniert. Das Insert eines resultierenden rekombinanten Moleküls (das entsprechende Plasmid wurde mit pBSPamHNL5g bezeichnet) wurde nach der oben beschriebenen Methode sequenziert und die Sequenz des klonierten Fragmentes war zu 100 % ident mit der Sequenz des oben beschriebenen mit den beiden Primern (1 & 2) erhaltenen PCR Produktes.

### Beispiel 3:

Sequenzanalyse des durch PCR Amplifikation mit den Primern 1 und 2 erhaltenen genomischen *Prunus amygdalus* DNA Fragmentes.

Im Bereich des durch PCR amplifizierten und sequenzierten DNA Abschnitts wurde ein offener Leserahmen gefunden, der durch 3 Introns unterbrochen ist. Diese drei Introns wurden mit mit Hilfe der Intron Consensus Sequenz "GT.....AG" identifiziert. Der Leserahmen beginnt mit einem ATG Codon bei Position +13 und endet mit einem Stop Codon bei Position +2151.
Für den kodierenden Bereich wurden die Fragmente von Position 13 bis 115 (Exon I), Position 258 bis 917 (Exon II), 1121 bis 1961 (Exon III) und 2078 bis 2150 (Exon IV) zusammengefügt. Die zusammengefügte DNA Sequenz kodiert für ein Protein mit 559 Aminosäuren und einem berechneten Molekulargewicht von 61 kDa, bzw. 57, 9 kDa für eine N-terminal prozessierte Form. Die Peptidmassen wurden mit Hilfe des Programmpakets GCG (Genetics Computer Group, Wisconsin, USA) berechnet. Für dieses Protein wurde die Bezeichnung *Pam*HNL5 festgelegt.
Die für den offenen Leserahmen (ohne Introns) abgeleitete Proteinsequenz ist in Figur 3 gezeigt. Es konnten ausgeprägte Homologien zu bekannten Hydroxynitrillyasen von *Rosaceae* festgestellt werden ("Blast" Programm, GCG Paket, Version 10.1, Genetics Computer Group, Wisconsin, USA) wobei die höchsten Homologien zu den publizierten Sequenzen von Mdl1 aus *Prunus amygdalus* (99 prozentige Identität, Suelves et al. 1998) und von Mdl5 aus *Prunus serotina* (94 prozentige Identität, Hu und Poulton, 1999) bestehen. Mit Hilfe dieser Homologie wurde auch eine spaltbare Signalsequenz mit Spaltung zwischen S27 und L28 identifiziert. Eine solche Spaltstelle konnte bei den beiden Isoenzymen Mdl1 und Mdl4 von *Prunus serotina* durch N-terminale Sequenzierung der aus Pflanzenmaterial gereinigten nativen Proteine nachgewiesen werden (Zihua Hu und Jonathan E. Poulton, Plant Physiology, 119, 1535 -1546, 1999). Die Sequenzen von verschiedenen in *Rosacea species* vorhandenen HNL Isoenzymen sind nur von *Prunus serotina* bekannt. Aufgrund der höchsten Homologien zur Sequenz von Mdl5 *aus Prunus serotina* wurde das neue *HNL* Gen aus *Prunus amygdalus* als *HNL*5 festgelegt. Die Suche nach Sequenzmotiven in der PROSITE-Sequenzmotiv Datenbank (GCG Paket, Version 10.1, Genetics Computer Group, Wisconsin, USA) ergab das Vorhandensein von 13 potentiellen N-Glykosilierungsstellen (in Figur 3 eingezeichnet).

### Beispiel 4:

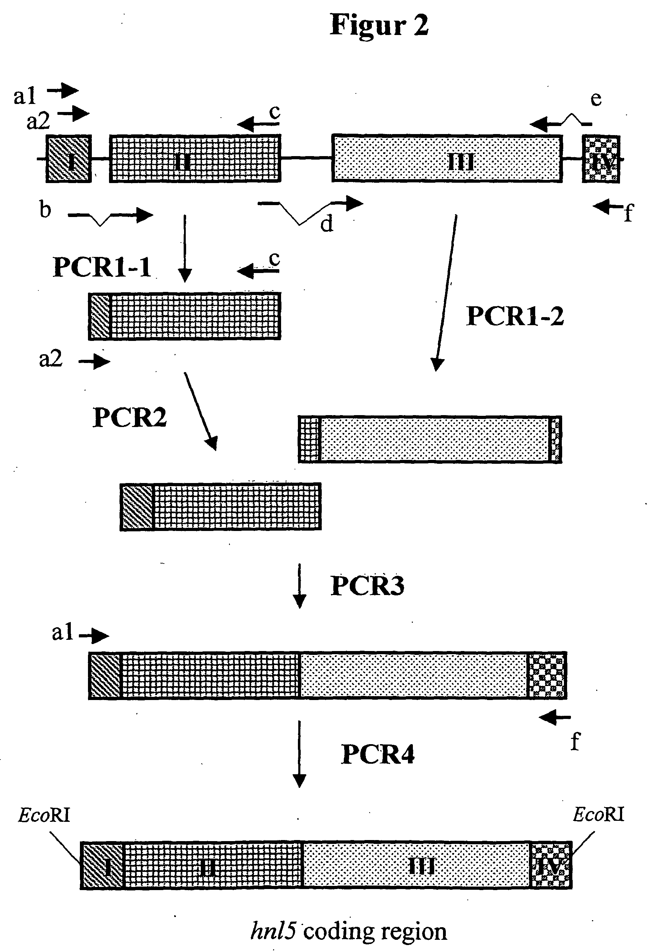

Gewinnung eines intronfreien *Prunus amygdalus HNL5* Gens durch PCR Splicing. Mittels einer speziellen PCR Strategie unter Verwendung von überlappenden Primern wurden die kodierenden Bereiche durch 4 aufeinanderfolgende PCR Reaktionen miteinander verbunden (entsprechend Figur 2).
In der ersten PCR Runde (PCR1-1 und PCR1-2) wurden die Exons II und III mit den Primerpaaren PamHNL5b/PamHNL5c (PCR1-1) bzw. PamHNL5d/PamHNL5e (PCR1-2) amplifiziert. Die 50 µl PCR Ansätze in 1x PCR Puffer (Qiagen) enthielten: je 100 pmol der entsprechenden Primer, 2,5 U "Hotstar" *Taq* DNA Polymerase (Qiagen), 5 µl eines dNTP (je 2 mM) Mix, 10 ng des Plasmids pBSPamHNL5g als Template. Es wurde folgendes Programm gefahren: 15 min 95 °C, 30 Zyklen 1 min 95 °C, 30 sec. 68°C, 1 min 72°C und zum Abschluss 5 min 72 °C zur Herstellung vollständiger Produkte). Die Produkte aus PCR1-1 und PCR1-2 wurden nach elektrophoretischer Trennung in einem Agarosegel mittels Qiaexll Kit aus dem Gel eluiert.
Durch Amplifikation von ca. 50 ng des Produktes aus PCR1-1 mit je 100 pmol der Primer PamHNL5a2 und PamHNL5c erfolgte in der zweiten PCR-Runde (PCR2) eine Verlängerung dieses ersten PCR Produktes. Es wurde folgendes Programm gefahren: 15 min 95 °C, 30 Zyklen 1 min 95 °C, 30 sec. 68°C, 1 min 72 °C und zum Abschluss 5 min 72 °C zur Herstellung vollständiger Produkte). Die sonstigen Bedingungen waren gleich wie bei PCR1. Dieses PCR Produkt wurde nach elektrophoretischer Trennung ebenfalls über ein Agarosegel gereinigt und eluiert

Durch primerlose PCR wurden in der dritten PCR-Runde (PCR3) die Produkte aus PCR1-2 und PCR2 mit Hilfe der überlappenden Enden miteinander verbunden (5 Zyklen mit 1 min bei 94°C, 30 sec bei 68°C und 1,5 min 72°C, je ca. 100 ng der beiden Produkte aus PCR1-1 und PCR2 in 50 µl Ansätzen in 1x PCR Puffer (Qiagen), 5 µl des dNTP (je 2 mM) Mix und 2,5 U "Hotstar" *Taq* DNA Polymerase (Qiagen).

Die Ergänzung zur vollen Länge des kodierenden *Prunus amygdalus HNL5* Gens und zur Amplifikation des vollständigen Produktes erfolgte in einer vierten PCR-Runde (PCR4) mit je 100 pmol der Primer PamHNL5a1 und PamHNL5f. Diese wurden direkt zum Reaktionsgemisch der PCR 3 zugegeben. Es wurde folgendes Programm gefahren: 20 Zyklen mit 1 min 95 °C, 30 sec. 63°C, 1,5 min 72 °C und zum Abschluss 5 min 72 °C). Die sonstigen Bedingungen waren gleich wie bei PCR1.

Das in der letzten PCR-Runde erhaltene Produkt wurde über ein präparatives Agarosegel aufgetrennt und die DNA mit einer Grösse von 1,6 - 1,8 kb mittels "Quiaexll" Kit (Qiagen) aus dem Gel eluiert und über die *Eco*RI Schnittstelle in das Plasmid pBSSK(-) einkloniert. Ein Klon mit korrektem Restriktionsmuster wurde ausgewählt und sequenziert.
Die Sequenz im kodierenden Bereich war zu 100 % ident mit den Exons der genomischen DNA Sequenz. Dieser Klon wurde als pBSPamHNL5orf bezeichnet.

Oligonukleotid-Primer

### Beispiel 5

Herstellen eines Basisfragments zur Konstruktion von Expressionsplasmiden für heterologe Expression des *Prunus amygdalus HNL5* Gens in Bakterien und Eukaryonten.

Ziel dieses Experiments war die Konstruktion eines Plasmides, aus dem ein für *Prunus amygdalus HNL5* kodierendes DNA Fragment für den Einbau in verschiedene Expressionsvektoren durch Ausschneiden mit Restriktionsendonukleasen gewonnen werden kann. Dabei wurden durch PCR Amplifikation über entsprechende Primer an den Enden des in pBSPamHNL5orf enthaltenen *Prunus amygdalus HNL5* Gens geeignete Sequenzen addiert.
Mit den Primern PCRHNL5-a und PCRHNL5-e wurde das Insert des Plasmids pBSPamHNL5orf mittels PCR amplifiziert (10 ng DNA des Plasmids pBSPamHNL5orf als Template, 400 ng Primer PCRHNL5-a, 200 ng Primer PCRHNL5-e). Die PCR Reaktion erfolgte in 50 µl Ansätzen in 1x PCR Puffer (Qiagen), enthaltend 5 µl des dNTP (je 2 mM) Mix und 1,2 U "Hotstar" *Taq* DNA Polymerase (Qiagen). Es wurde folgendes Programm gefahren: 15 min 95 °C, 30 Zyklen 1 min 95 °C, 30 sec. 68°C, 1,5 min 72 °C und zum Abschluss 5 min 72 °C zur Herstellung vollständiger Produkte.
Das erhaltene DNA Fragment wurde nach Schneiden mit der Restriktionsendonuklease *Eco*RI in den Vektor pBSSK(-) (Stratagene, USA) einkloniert und durch Sequenzierung verifiziert. Das resultierende Plasmid wurde pBSPamHNL5ex benannt.
Oligonukleotid-Primer:

### Beispiel 6

### Konstruktion von Expressionskonstrukten zur heterologen Expression des HNL5 Gens in Pichia pastoris.

DNA des Plasmids pBSPamHNL5ex wurde mit *Eco*RI geschnitten und das *HNL-*Fragment vom Vektoranteil mittels präparativer Gelelektrophorese getrennt. Nach Elution der *HNL*-Fragment DNA mittels Qiaex II Kit (Qiagen) wurde dieses über die *Eco*Rl Schnittstellen in die Plasmide pHILD2 und pGAPZ (Invitrogen, San Diego, CA, USA) einkloniert. Die richtige Orientierung der Inserts zu den Promotoren wurde mit Hilfe von Kontrollschnitten überprüft. Jeweils ein Klon mit einem korrekt orientierten Insert wurde ausgewählt und konserviert. Die korrekten Übergänge vom Vektoranteil zum eingebauten *HNL*-Fragment wurden durch Sequenzierung verifiziert. Diese beiden entstandenen Expressionsplasmide für *Pichia pastoris* wurden als pHILDPamHNL5a (für induzierbare Expression) und pGAPPamHNL5a (für konstitutive Expression) bezeichnet.

### Beispiel 7

### Induzierbare Expression des Prunus amygdalus HNL5 Gens in Pichia pastoris

DNA des Plasmids pHILDPamHNL5a wurde mit der Restriktionsendonuklease *Not*l geschnitten und in *Pichia pastoris* GS115 (Invitrogen, San Diego, CA, USA) transformiert. Die Transformation erfolgte nach den Vorschriften des "Pichia Expression Kit" (Invitrogen, San Diego, CA, USA). 100 Histidin-prototrophe Klone wurden nach den Vorschriften des "Pichia Expression Kit" in Flüssigmedium (500 ml Schüttelkulturen) angezüchtet und 48 Stunden mit Methanol induziert. Die Zellen wurden abzentrifugiert und mit Aufschlusspuffer (50 mM Tris-HCL, pH 7,6) zu einer optischen Dichte (OD₆₀₀) von 50.0 suspendiert und nach der "Glaskugelmethode" (Ausubel et al., Current Protocolls in Molecular Biology, Green Publishing Associates and Wiley-Interscience, New York, 1999) in einem "Merckenschlager" Homogenisator (Fa. Braun, Melsungen, BRD) aufgeschlossen. Sowohl die Kulturüberstände als auch die Zelllysate wurden auf HNL-Enzym Aktivität mit einem Standard Aktivitätsassay (analog WO 97/03204) unter Verwendung von racemischem Mandelonitril als Substrat bestimmt. Zwei Klone, die bei diesen Schüttelkolbenexperimenten die besten Aktivitäten im Kulturüberstand zeigten (*Pichia pastoris* PamHNL5-a37 und *Pichia pastoris* PamHNL5-a4) wurden ausgewählt und konserviert. Eine Analyse des Methanolverwertungstyps ergab den Phänotyp Mut⁺ (gute Verwertung von Methanol) für *Pichia pastoris* PamHNL5-a37 und den Phänotyp Mut^{s} (langsame Verwertung von Methanol) für *Pichia pastoris* PamHNL5-a4.

### Beispiel 8

### Konstitutive Expression des Prunus amygdalus HNL5 Gens in Pichia pastoris

Das Plasmid pGAPPamHNL5a wurde in P. pastoris GS115 transformiert. Die Transformation erfolgte nach den Vorschriften des Pichia Expression Kit der Invitrogen Corp (San Diego, CA, USA). Die Selektion von Transformanten erfolgte auf YPD Vollmediumplatten mit 100 mg/l Zeocin. 100 Zeocin-resistente Klone wurden in je 500 ml YPD Vollmedium angezüchtet und 96 Stunden bei 30 °C geschüttelt. Die Zellen wurden abzentrifugiert und die HNL Aktivität im Kulturüberstand bestimmt. Ein Klon der die beste Aktivität im Kulturüberstand zeigte wurde konserviert und mit *Pichia pastoris* PamHNL5-a2 bezeichnet.

### Beispiel 9

Produktion von HNL mit einem mit dem *HNL5* Gen aus *Prunus amygdalus* transformierten Stamm von *Pichia pastoris* (durch Methanol indizierbares Expressionssystem) im Laborbioreaktor.

Die Züchtung von *Pichia pastoris* PamHNL5-a37 erfolgte in einem Standard-Laborbioreaktor (42 L Gesamtvolumen) in einem dreiphasigen Verfahren. Dieses bestand aus einer ersten exponentiellen und einer zweiten linearen Wachstumsphase zur Bildung von Biomasse und einer anschließenden Expressionsphase zur Bildung des rekombinanten *Prunus amygdalus* HNL Enzyms, wobei im Prinzip nach der für die Produktion von rekombinanter *Hevea brasiliensis* HNL beschriebenen Methode (Hasslacher, M., Schall, M., Hayn, M., Bona, R., Rumbold, K., Lückl, J., Griengl, H., Kohlwein, S.D., Schwab, H.: High level intracellular expression of hydroxynitrile lyase from the tropical rubber tree *Hevea brasiliensis* in microbial hosts. Protein Expression and Purification **11**, 61-71, 1997) vorgegangen wurde.
Im Detail wurden folgende Bedingungen eingehalten:
Die Chemikalien 1.-8., bemessen für 20 Liter wurden in 15 Liter Wasser gelöst im Bioreaktor vorgelegt und zusammen mit dem Reaktor bei 121 °C für 1 Stunde sterilisiert. Nach Abkühlung auf 29°C wurde der pH Wert im Medium mit Ammoniak (Chemikalium 9, in steriler Zulaufflasche vorgelegt) auf pH 5,0 eingestellt. Danach wurden ca. 200 ml einer steril filtrierten Spurenelemente-Lösung (Chemikalien 10-18, entsprechende Mengen für 20 L) über eine Zulaufflasche in den Bioreaktor eingebracht.
Der so vorbereitete Bioreaktor wurde mit 2 Liter Vorkultur, die in Schüttelkolben bei 30°C nach den im Handbuch des "Pichia Expression Kit" (Invitrogen Corp., San Diego, CA, USA) angegebenen Bedingungen angezüchtet wurde, beimpft. Die Kultivierung wurde bei einer konstanten Temperatur von 29°C durchgeführt. Durch Regelung der Belüftung (zwischen 15 und max. 40 Lit Luft/min) und der Rührerdrehzahl (zwischen 250 bis 500 Upm) wurde der O₂-Partialdruck auf einem Wert von größer als 30% der Sättigungskonzentration gehalten. Nach 21 Stunden war die Biomasse auf einen Wert im Bereich von 22 g/L Zelltrockengewicht (ZTG) angewachsen. Ab diesem Zeitpunkt wurde steriles Glycerin in konstanten kleinen Portionen in Abständen von 15 min zudosiert, wobei pro Stunde 130 g Glycerin zugesetzt wurden. In dieser zweiten linearen Wachstumsphase konnte in einem Zeitraum von 42 Stunden eine Biomassekonzentration im Bereich von 70 g/L ZTG erreicht werden.
Danach wurde die dritte Phase mit der Induktion der Expression durch Zudosierung von Methanol eingeleitet. Dabei wurde der Methanolgehalt in der Kulturbrühe auf einen Wert von 0,8-1 Gewicht % eingestellt. Zu Induktionsbeginn und nach zwei Tagen Induktion wurde jeweils eine weitere Portion sterile Spurenelementelösung (Chemikalien 10-18, entsprechende Mengen für 20 L, gelöst in ca. 200 ml Wasser) zugegeben. Nach 110 Stunden Induktionsphase konnte eine Enzymmenge von 110 U/ml Kulturbrühe erhalten werden. Nach Abtrennen der Zellen durch beispielsweise Zentrifugation kann eine Rohenzympräparation erhalten werden, die direkt für biokatalytische Umsetzungen eingesetzt werden kann.

Folgende Chemikalien wurden zur Herstellung des Kulturmediums benutzt (Menge pro Liter):

| | |
|---|---|
| 1.85% ortho-Phosphorsäure | 21 ml |
| 2. CaSO₄ | 0,9 g |
| 3. K₂SO₄ | 14,3 g |
| 4. MgSO₄.7H₂O | 12,2 g |
| 5. KOH (Chemikalien 1 bis 5 in p.a. Qualität) | 3,3 g |
| 6. Glycerin, technische Qualität. | 50 ml |
| 7. Entionisiertes Wasser, Hausqualität, Leitfähigkeit | 5,5-9,1 µS/cm |
| 8. Antischaummittel 10 % Acepol 83E (Carl Becker Chemie GmbH, Hamburg, Deutschland | 1ml |
| 9. 25%Ammoniak, technische Qualität | |

Spurenelemente und Vitamin H (alle Chemikalien in p.a. Qualität) :

| | |
|---|---|
| 10. Biotin | 0,8 mg |
| 11. CuSO₄.5H₂O. | 24,0 mg |
| 12. Kl | 0,32 mg |
| 13.MnSO₄.H₂O. | 12,0 mg |
| 14. Na₂MoO₄.2 H₂O | 0,2 mg |
| 15.H₃BO₃ | 0,08 mg |
| 16. CoCl₂ | 2,0 mg |
| 17. ZnSO₄.7H₂O | 80 mg |
| 18. Fe(II)SO₄.7H₂O | 260 mg |

### Beispiel 10:

Rekombinantes Protein mit HNL-Aktivität, das mit dem rekombinanten Stamm *Pichia pastoris* PamHNL5-a37 wie in Beispiel 9 beschrieben produziert wurde, wurde einer Analyse der Glykosylierung durch Verdau mit Endoglykosidasen unterzogen. Dazu wurden 100 ml des Kulturüberstandes durch Ultrafiltration (Biomax 30,000 NMWL, Millipore, Bedford, MA, USA) 10 fach konzentriert. Die Proben 1-2 wurden mit N-Glycosidase F (N-Glykosidase F Kit, Roche Diagnostics, Mannheim, D) behandelt.
Bei den Proben 4-5 wurde eine HNL Präparation aus Mandeln der Fa. Roche verwendet und bei den Proben 6 und 7 wurde der aufkonzentrierte Kulturüberstand mit Endoglykosidase H (Roche, Diagnostics, Mannheim, D) behandelt. Alle Ansätze wurden in 10 µl Gesamtvolumen durchgeführt.
- Std.: Molekulargewichtsstandard aus dem N-Glykosidase F Kit (5 µl =5 µg)
- Probe 1:: 2 U *Pam*HNL5 mit 2,4 U Enzym nach Vorschrift des Kits behandelt.
- Probe 2:: 2 U *Pam*HNL5 mit 2,4 U Enzym nach Vorschrift des Kits jedoch ohne Denaturierungspuffer und ohne Hitzedenaturierung behandelt
- Probe 3:: 2 *Pam*HNL5 ohne Behandlung
- Probe 4:: 0,25 U der Roche R-HNL Präparation grade III aus Mandeln (10,3 U/mg) behandelt mit 2,4 U N-Glycosidase F nach Vorschrift des Kits
- Probe 5:: 0,25 U der Roche Präparation grade III (10,3 U/mg) unbehandelt
- Probe 6: 2,4 U *Pam*HNL5 wurden mit 50 mU Endoglykosidase H in 20 mM Phosphatpuffer, ohne Denaturierung für 12 Stunden bei 37°C inkubiert.
- Probe 7: 2,4 U *Pam*HNL5 wurden mit 5 mU Endoglykosidase H in 20 mM Phosphatpuffer, 0,2% SDS, 0,4 % Merkaptoethanol für 12 Stunden bei 37°C
inkubiert.

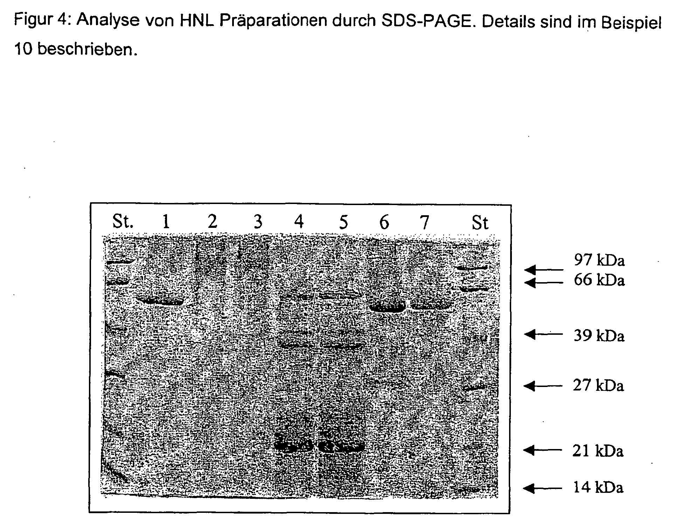

Nach den Behandlungen mit den Glykosidasen wurden die Proben auf einem 12-prozentigen SDS-Polyacrylamid Gel getrennt und mit Commassie Blue gefärbt.
Diese Ergebnisse (siehe Figur 4) zeigen, dass ein Grossteil der an *Pam*HNL5 gebundenen Oligosaccharide durch Endoglykosidase H auch ohne Denaturierung des *Pam*HNL5 Proteins entfernt werden kann.
Durch die Abspaltung der Oligosaccharide entsteht aus einem bei Größen von 70 bis über 100 kDa sichbaren Proteinschmier eine scharfe Bande bei etwa 60 kDa, was dem berechneten Molekulargewicht eines nicht glykosylierten *Pam*HNL5 Proteins entspricht.
Eine vergleichbare Proteinbande ist im Roche Präparat nicht oder nur in stark untergeordnetem Ausmass vorhanden. Außerdem kann kein signifikanter Unterschied zwischen einer unbehandelten Proteinpräparation und einer mit Endoglykosidase F behandelten Präparation gesehen werden. Aus diesem Befund kann eindeutig festgestellt werden, dass sich das rekombinante *Pam*HNL5 Enzym komplett vom aus Mandelkernnen gewonnenen Enzymmaterial unterscheidet.

### Beispiel 11:

0,5 g (3,9 mmol) Octanal wurden in 6 ml tert-Butylmethylether gelöst und mit 7,5 ml einer wäßrigen Enzymlösung mit rekombinanter R-HNL aus Beispiel 9 (pH 3,8) versetzt. Nach Zugabe von 0,33 ml (8,4 mmol) Blausäure wurde zur Bildung einer Emulsion am Magnetrührer bei Raumtemperatur heftig gerührt und die Reaktion mittels GC an einer Cyclodextrinsäule verfolgt. Nach 3 Stunden Reaktionszeit wurde Cyanhydrin mit 81,1 %ee und 48 % Umsatz gebildet.

### Beispiel 12:

80 ml (34 units / mmol Aldehyd) einer wäßrigen Enzymlösung mit rekombinanter R-HNL (34 units / mmol Aldehyd) wurden mit 10 ml 200 mM Kaliumphosphat / Natriumcitratpuffer pH 3,8 verdünnt und in eine auf 10 °C vorgekühlte Lösung aus 42,2 g (300 mmol) 2-Chlorbenzaldehyd und 42 ml tert-Butylmethylether gegeben. Anschließend wurden unter Rühren mit 950 rpm 19,6 ml (501 mmol) Blausäure innerhalb von 40 min in die Reaktionsmischung dosiert. Der Reaktionsverlauf wurde, nach Derivatisierung des Cyanhydrins mit Acetylchlorid, mittels GC an einer Cyclodextrinsäule verfolgt.

| Stunden | % Umsatz | % ee |
|---|---|---|
| 3,5 | 71,5 | 90,6 |
| 22 | 99,7 | 90 |

### Vergleichsbeispiel :

0,25 bis 1 ml R-OxynitrilaseLösung (E.C.4.1.2.10; 2187 units/ml) wurden mit 50 mM Citrat/Phosphatpuffer (pH 4,0) auf 4 ml verdünnt und der pH-Wert der Enzymlösung gegebenenfalls mit einigen Tropfen Citronensäurelösung auf pH 4,0 eingestellt. Zu dieser Lösung wurde eine Lösung von 3 ml tert.-Butylmethylether und 0,8g (5,69 mmol) 2-Chlorbenzaldehyd zugegeben und anschließend 445 µl (11,38 mmol) Blausäure zugefügt. Die Reaktionsmischung wurde mittels Magnetrührer bei Raumtemperatur mit 900 rpm gerührt.

Der Umsatz und die Enantiomerenreinheit des gebildeten (R)-Cyanhydrins wurde mittels GC analysiert.
Dazu wurde eine Probe der Reaktionslösung zentrifugiert und 50 µl der organischen Phase mit Dichlormethan verdünnt. Nach einer Derivatisierung mit Acetylchlorid wurde auf einer Cyclodextrinsäule gaschromatografisch analysiert.

| Zeit (h) | 0,25 ml Enzyml entspricht 96 units / mmol Aldehyd | | 0,5 ml Enzyml entspricht 192 units / mmol Aldehyd | | 1,0 ml Enzyml entspricht 384 units / mmol Aldehyd | |
|---|---|---|---|---|---|---|
| | % Umsatz | %ee | % Umsatz | %ee | %Umsatz | %ee |
| 1,5 | 79,1 | 77,5 | 97,6 | 81,6 | 98,7 | 89,4 |
| 3 | 98 | 77,4 | 100 | 81,5 | 100 | 89,1 |

Der Vergleichsversuch zeigte, dass bei Verwendung der erfindungsgemäßen rekombinanten Proteine analog Beispiel 11 lediglich ein Zehntel der benötigten Menge an nativem Protein benötigt wird, um vergleichbare Enantiomerenreinheiten (ee-Werte) zu erzielen.

## Patentansprüche

1. Isolierte Nukleinsäure, welche eine Nukleinsäure mit der Nukleotidsequenz SEQ ID NO: 19 von Nukleotid 13 bis Nukleotid 2151 durchgehend oder diese Sequenz abzüglich der Intron-Bereiche von den Nukleotiden 116 bis 257, 918 bis 1120 und 1962 bis 2077 umfasst.

2. Rekombinantes Protein, herstellbar durch heterologe Expression der DNA-Sequenz gemäß Anspruch 1 in geeigneten Wirtszellen.

3. Rekombinantes Protein nach Anspruch 2, **dadurch gekennzeichnet, dass** es eine wirtsspezifische Glykosylierung aufweist.

4. Rekombinantes Protein nach Anspruch 2, **dadurch gekennzeichnet, dass** es durch Expression in einem eukaryontischen Mikroorganismus hergestellt wird.

5. Rekombinantes Protein nach Anspruch 2, **dadurch gekennzeichnet, dass** es durch Expression in einem Pilz hergestellt wird.

6. Rekombinantes Protein nach Anspruch 2, **dadurch gekennzeichnet, dass** das Protein die aus der Nukleotidsequenz des Gens nach Anspruch 1 abgeleitete Aminosäuresequenz SEQ ID NO:20 aufweist

7. Verwendung von Proteinen nach einem der Ansprüche 2-6 zur Herstellung von (S)- oder (R)-Cyanhydrinen.

8. Verfahren zur Herstellung von (S)- oder (R)-Cyanhydrinen, **dadurch gekennzeichnet, dass** aliphatische, aromatische oder heteroaromatische Aldehyde und Ketone in Anwesenheit eines Cyanidgruppendonors mit Proteinen nach einem der Ansprüche 2-7 im organischen, wässrigen oder 2-Phasensystem oder in Emulsion umgesetzt werden.

## Claims

1. Isolated nucleic acid which comprises the nucleotide sequence SEQ ID NO: 19 from nucleotide 13 right through to nucleotide 2151 or this sequence minus the intron regions of nucleotides 116 to 257, 918 to 1120 and 1962 to 2077.

2. Recombinant protein, which can be prepared in suitable host cells by heterologous expression of the DNA sequence according to Claim 1.

3. Recombinant protein according to Claim 2, **characterized in that** it comprises host-specific glycosylation.

4. Recombinant protein according to Claim 2, **characterized in that** it is prepared by expression in a eukaryotic microorganism.

5. Recombinant protein according to Claim 2, **characterized in that** it is prepared by expression in a fungus.

6. Recombinant protein according to Claim 2, **characterized in that** the protein has the amino acid sequence SEQ ID NO: 20 derived from the nucleotide sequence of the gene according to Claim 1.

7. Use of proteins according to any of Claims 2-6 for preparing (S)- or (R)-cyanohydrins.

8. Process for preparing (S)- or (R)-cyanohydrins, **characterized in that** aliphatic, aromatic or heteroaromatic aldehydes and ketones are reacted with proteins according to any of Claims 2-7 in an organic, aqueous or 2-phase system or in emulsion in the presence of a cyanide group donor.

## Revendications

1. Acide nucléique isolé, qui comprend une séquence nucléique avec la séquence nucléotidique SEQ ID n°19 allant du nucléotide 13 au nucléotide 2151 ou cette séquence moins les domaines des introns des nucléotides 116 à 257, 918 à 1120 et 1962 à 2077.

2. Protéine recombinante, pouvant être obtenue par expression hétérologue de la séquence d'ADN selon la revendication 1 dans des cellules hôtes adéquates.

3. Protéine recombinante selon la revendication 2, **caractérisée en ce qu'**elle présente une glycosylation hôte-spécifique.

4. Protéine recombinante selon la revendication 2, **caractérisée en ce qu'**elle est fabriquée par expression dans un microorganisme eucaryote.

5. Protéine recombinante selon la revendication 2, **caractérisée en ce qu'**elle est fabriquée par expression dans un champignon.

6. Protéine recombinante selon la revendication 2, **caractérisée en ce que** la protéine, qui dérive de la séquence nucléotidique du gène selon la revendication 1, présente la séquence d'acides aminés SEQ ID n°20.

7. Utilisation de protéines selon l'une des revendications 2-6 pour la fabrication de (S)- ou (R)-cyanhydrines.

8. Procédé de fabrication de (S)- ou (R)-cyanhydrines, **caractérisé en ce que** des aldéhydes et cétones aliphatiques, aromatiques ou hétéroaromatiques sont utilisés en présence d'un donneur de groupe cyanure avec des protéines selon l'une des revendications 2-7 dans un système à phase double, aqueux ou organique ou en émulsion.
